# NEW EUROPEAN PATENT SPECIFICATION

(11) **EP 3 137 060 B2**
(45) Date of publication and mention of the opposition decision: **20.12.2023**
(45) Mention of the grant of the patent: 11.11.2020
(21) Application number: 15786792.0
(22) Date of filing: 01.05.2015
(51) Int. Cl.: A61K 9/10, A61K 47/38, A61K 47/36, A61K 47/26, A61K 9/50, A61K 31/155, A61K 9/14, A61K 31/165, A61K 31/17, A61K 31/43, A61K 31/4439, A61K 31/522, A61P 1/04, A61P 3/10, A61P 9/12, A61P 25/14, A61P 31/04, A61P 31/12, A61K 31/132

(54) **EXTENDED RELEASE SUSPENSION COMPOSITIONS**
SUSPENSIONSZUSAMMENSETZUNGEN MIT VERLÄNGERTER FREISETZUNG
COMPOSITIONS EN SUSPENSION À LIBÉRATION PROLONGÉE

(30) Priority: 01.05.2014 IN 1183DE2014
(43) Date of publication of application: 08.03.2017
(73) Proprietor: Sun Pharmaceutical Industries Ltd, 400063 Mumbai, Maharashtra (IN)
(72) Inventor: KUMAR, Ashish, Jhajjar Haryana 124507 (IN); SHEAR, Rajesh Srikrishan, Gurgaon Haryana 122001 (IN); JAIN, Satish Kumar, Bilaspur Chhattisgarh 495001 (IN); SINGH, Romi, Barat, Varanasi Uttar Pradesh 221002 (IN); JAIN, Paras P., Amravati Maharashtra 444708 (IN)
(74) Representative: HGF
(86) International application number: PCT/IB2015/053209
(87) International publication number: WO 2015/166473

(56) References cited:
- WO-A1-2004/012715
- WO-A1-2006/030297
- WO-A1-2006/086856
- WO-A2-2010/045656
- FR-A1- 2 897 267
- US-A- 5 955 106
- US-A1- 2006 165 807
- US-A1- 2010 092 562

## Description

### Field of the Invention

The present invention relates to extended release suspension compositions of metformin or guanfacine. Said extended release suspension compositions comprise multiple coated cores of metformin or guanfacine and a suspension base, wherein the suspension base generates a hypertonic condition such that there is no substantial change in the in-vitro dissolution release profile of the extended release suspension compositions upon storage for at least seven days. The invention also relates to processes for the preparation of said extended release suspension compositions.

### Background of the Invention

Extended release solid compositions are preferred dosage forms over immediate release solid compositions, especially for active ingredients showing fluctuations in the plasma concentration and for active ingredients having short half-lives. Extended release solid compositions can be in the form of tablets or capsules, wherein the release of the active ingredient is controlled by using a reservoir or a matrix system. However, extended release solid compositions suffer from certain drawbacks such as difficulty in swallowing, particularly for certain groups of patients, e.g., pediatrics and geriatrics, resulting in poor patient compliance. Further, high doses of active ingredient lead to large-sized compositions which aggravates this problem. Also, there remains a tendency to divide extended release solid compositions such as tablets into small pieces in order to facilitate administration, which may ultimately lead to inaccurate dosing and/or dose dumping. In view of all this, extended release liquid compositions provide the best alternative over extended release solid compositions. Extended release liquid compositions are easy to administer, thereby leading to enhanced patient compliance. Additionally, extended release liquid compositions provide a unique advantage of having a flexible dosing regimen.

Although extended release liquid compositions are advantageous, there remain some complexities involved in formulating such compositions. The important prerequisite of these compositions is to provide the desired extended release of the active ingredient throughout its shelf life, as irregular release may lead to sub-therapeutic or toxic effects. The key hurdle remains to avoid the release of the active ingredient into the suspension base during storage, and to allow release only when the suspension enters the stomach.

The prior art discloses various approaches for the preparation of extended release liquid compositions.

U.S. Patent No. 6,156,340 discloses a controlled release suspension comprising inert cores coated with an active ingredient, which were further coated with two layers of polymers with increased permeability to water.

U.S. Patent No. 7,906,145 discloses a sustained release suspension of microcapsules in an aqueous liquid phase, wherein each microcapsule comprises a core of an active ingredient and a film coating applied to the core which controls the modified release of the active ingredient in gastrointestinal fluids, comprising a film-forming polymer, a nitrogen-containing polymer, a plasticizer, and a surfactant/lubricant.

PCT Publication No. WO 2011/107855 discloses a ready to use sustained release oral suspension comprising inert pellets surrounded by a seal coating, an active ingredient layer surrounding the seal coated inert pellets, and a coating layer comprising a rate-controlling polymer surrounding the active ingredient layer.

PCT Publication No. WO 2011/077451 discloses a controlled release suspension comprising an active ingredient loaded core and a polymer dispersion comprising a controlled-release polymer, wherein said suspension has a duration of therapeutic effect for at least about 6 hours to about 30 hours after oral administration.

PCT Publication No. WO 2008/122993 discloses a suspension of an active ingredient containing microparticles with at least one coat of a pH-independent polymer.

PCT Publication No. WO 2012/063257 and U.S. Publication No. 2008/0118570 disclose controlled release suspensions employing ion-exchange resins.

In the formulations disclosed in the prior art, there is a possibility of leaching of the active ingredients from the coated units into the media during storage. Further, although ion-exchange resin systems provide the desired extended release without significant leaching during storage, these systems require chemical binding of the active ingredient to the resin, which is complicated and not suitable for many active ingredients.

There remains a need in the art to formulate extended release liquid compositions of active ingredients which are based on a simplified technology and which provide the desired extended release throughout the shelf life of the compositions. The present invention provides such compositions and improves patient compliance by reducing dosing frequency for pediatric as well as geriatric patients.

The extended release suspension compositions of the present invention are relatively simple, easy to manufacture, and functionally reproducible. The extended release suspension compositions provide the desired extended release throughout the shelf life of the compositions. The scientists of the present invention have surprisingly discovered that a hypertonic condition generated in the suspension base affects the leaching of the active ingredient from the extended release coated cores into the suspension base. This hypertonic condition minimizes the leaching problem and thus provides substantially similar *in-vitro* extended release of the active ingredient throughout the shelf life of the compositions. This consistent *in-vitro* release then ensures a steady plasma concentration with no fluctuations throughout the shelf life of the compositions. Additionally, the extended release suspension compositions of the present invention are able to incorporate two or more active ingredients with different release profiles or two or more incompatible active ingredients in a single composition.

### Summary of the Invention

The present invention relates to extended release suspension compositions of metformin or guanfacine. Said extended release suspension compositions comprise multiple coated cores of metformin or guanfacine and a suspension base, wherein the suspension base generates a hypertonic condition such that such that there is no substantial change in the in-vitro dissolution release profile of the extended release suspension compositions upon storage for at least seven days. The invention also relates to processes for the preparation of said extended release suspension compositions.

The extended release suspension compositions of the present invention are easy to administer, thereby leading to enhanced patient compliance. Further, said extended release suspension compositions provide better dose flexibility depending on the age and body weight of the patient. Also, said extended release suspension compositions are stable, easy to commercially manufacture, and provide reproducible bioavailability. Additionally, said extended release suspension compositions provide a pleasant mouth feel, thereby further enhancing patient compliance.

### Brief Description of the Drawings

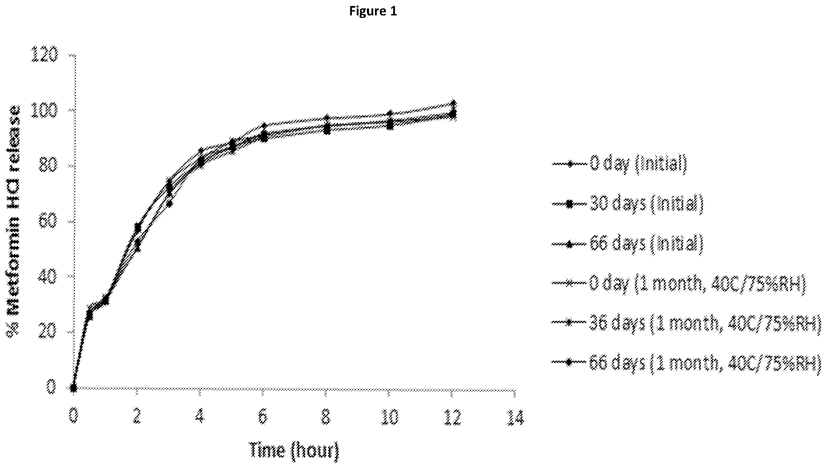

Figure 1 shows the *in-vitro* dissolution release on day 0, day 30, and day 66 of the extended release suspension composition prepared according to Example 4 upon storage at room temperature. This figure also shows the *in-vitro* dissolution release on day 0, day 36, and day 66 of the extended release suspension composition (at room temperature) formed after reconstituting the powder stored for one month at accelerated conditions.

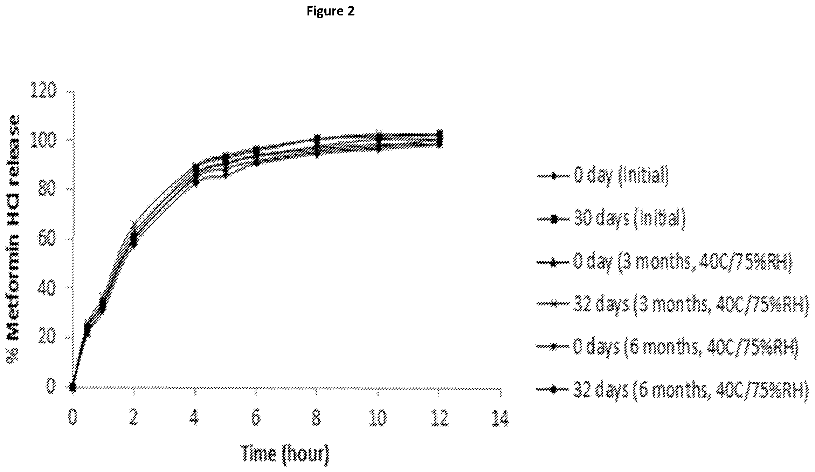

Figure 2 shows the *in-vitro* dissolution release on day 0 and day 30 of the extended release suspension composition prepared according to Example 5 upon storage at room temperature. This figure also shows the *in-vitro* dissolution release on day 0 and day 32 of the extended release suspension composition (at room temperature) formed after reconstituting the powder stored for three months and six months at accelerated conditions.

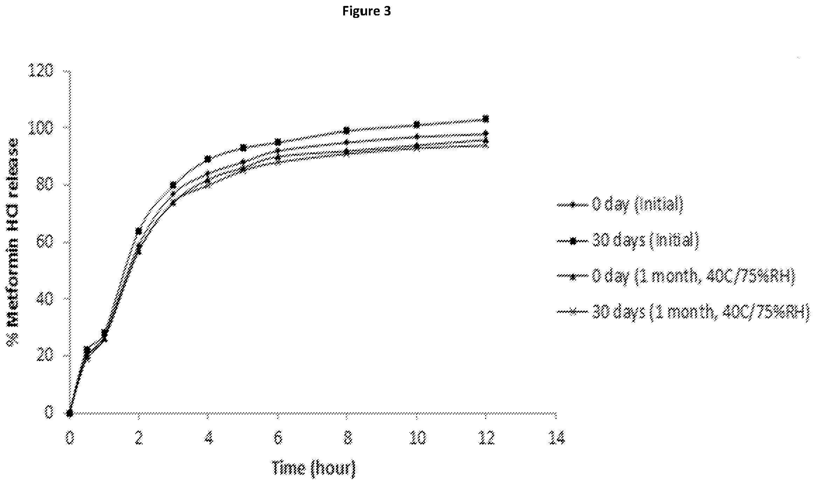

Figure 3 shows the *in-vitro* dissolution release on day 0 and day 30 of the extended release suspension composition prepared according to Example 6 upon storage at room temperature. This figure also shows the *in-vitro* dissolution release on day 0 and day 30 of the extended release suspension composition (at room temperature) formed after reconstituting the powder stored for one month at accelerated conditions.

### Detailed Description of the Invention

A first aspect of the present invention provides an extended release suspension composition comprising:
- (a) multiple coated cores comprising:
   ∘ (i) a core comprising an active ingredient; and
   ∘ (ii) a coating layer over said core comprising one or more release-controlling polymers;
- (b) a suspension base comprising an osmogent, wherein the suspension base generates a hypertonic condition such that there is no substantial change in the in-vitro dissolution release profile of the extended release suspension compositions upon storage for at least seven days,
wherein the active ingredient is selected from the group comprising metformin or guanfacine.

According to another embodiment of the above aspect, the suspension base has an osmolality of not less than about 1 osmol/kg of the suspension base, and in particular not less than about 2 osmol/kg of the suspension base. In accordance with the invention, the suspension base comprises an osmogent.

According to another embodiment of the above aspect, the extended release suspension composition is in the form of a suspension or a reconstituted powder for suspension.

According to another embodiment of the above aspect, the release-controlling polymer is selected from the group comprising a pH-dependent polymer, a pH-independent polymer, or mixtures thereof.

According to another embodiment of the above aspect, the core is in the form of a bead, a pellet, a granule, a spheroid, or the like.

According to another embodiment of the above aspect, the active ingredient is layered onto an inert particle to form the core.

According to another embodiment of the above aspects, the composition is a suspension or a reconstituted powder for suspension.

The term "extended release," as used herein, refers to the release profile of the active ingredient over an extended period of time, *e.g*., over a period of 4, 6, 8, 12, 24 hours, or more.

The term "hypertonic condition," as used herein, means the suspension base has higher solute concentration which helps to generate high osmotic pressure such that there is no leaching of active ingredient from the coated cores into the suspension base. In the present invention, the solutes are osmogents *i.e*., pharmaceutically acceptable inert water-soluble compounds that contribute towards generating hypertonic conditions in the suspension base.

The term "osmolality ratio," as used herein, means the ratio of the osmolality of the external phase to the osmolality of the internal phase. The external phase herein means the suspension base without multiple coated cores of the active ingredient. The internal phase herein means the coated cores of the active ingredient. As the direct measurement of the osmolality of the internal phase *i.e.,* coated cores is difficult, the osmolality of the internal phase herein, is represented as the osmolality of a solution which prevents significant leaching of the active ingredient from the coated cores into the solution. The leaching of the active ingredient from the coated cores is determined by the difference in the osmolalities across the coating layer and the absence of any significant leaching from the coated cores directs that the osmolality of the solution has become equal to the osmolality of the coated cores. The osmolality ratio of the extended release suspension compositions of present invention is at least about 1.

The term "osmolality," as used herein, is expressed as number of moles of any water-soluble compound per kg of a liquid phase. The liquid phase can be a suspension base or a solution. In the present invention, the osmolality may be measured according to known methods, such as using a vapor pressure osmometer, a colloid osmometer, or a freezing point depression osmometer such as Osmomat 030-D or Osmomat 3000, in particular by a freezing point depression osmometer.

The osmolality of the suspension base of the extended release suspension compositions of the present invention remains equivalent upon storage for at least seven days. Particularly, the osmolality of the suspension base measured after one month remains equivalent to the osmolality of the suspension base measured as soon as practicable after preparation of the extended release suspension compositions. More particularly, the osmolality of the suspension base measured after three months or six months remains equivalent to the osmolality of the suspension base measured as soon as practicable after preparation of the extended release suspension compositions. The equivalent osmolality of the suspension base ensures that there is no leaching of the active ingredient from the coated cores into the suspension base.

The term "stable," as used herein, refers to chemical stability, wherein not more than 5% w/w of total related substances are formed on storage at 40°C and 75% relative humidity (R.H.) or at 25°C and 60% R.H. for a period of at least three months to the extent necessary for the sale and use of the composition.

The term "inert particle," as used herein, refers to a particle made from a sugar sphere also known as a non-pareil seed, a microcrystalline cellulose sphere, a dibasic calcium phosphate bead, a mannitol bead, a silica bead, a tartaric acid pellet, a wax based pellet, and the like.

The term "about," as used herein, refers to any value which lies within the range defined by a variation of up to ±10% of the value.

The term "equivalent" as used herein, refers to any value which lies within the range defined by a variation of up to ±30% of the value.

The term "significant leaching," as used herein means more than 20% of the active ingredient is leached out from the coated cores into the solution.

The *in-vitro* dissolution release profile of the extended release suspension compositions of the present invention upon storage for at least seven days remains substantially similar to the initial *in-vitro* dissolution release profile obtained as soon as practicable after preparation of the extended release suspension compositions. Particularly, the *in-vitro* dissolution release profile of the extended release suspension compositions of the present invention upon storage for at least one month remains substantially similar to initial *in-vitro* dissolution release profile obtained as soon as practicable after preparation of the extended release suspension compositions. More particularly, the *in-vitro* dissolution release profile of the extended release suspension compositions of the present invention upon storage for at least three months remains substantially similar to initial *in-vitro* dissolution release profile obtained as soon as practicable after preparation of the extended release suspension compositions. More particularly, the *in-vitro* dissolution release profile of the extended release suspension compositions of the present invention upon storage for at least six months remains substantially similar to initial *in-vitro* dissolution release profile obtained as soon as practicable after preparation of the extended release suspension compositions. In the present invention, wide ranges of dissolution methodologies can be utilized for different active ingredients. These methodologies can be adopted to vary in hydrodynamic mechanism to simulate *in-vivo* conditions by using different dissolution apparatuses, volume of media, pH of media ranging from 1.0 to 7.5, any standard USP buffers with standard molarity, addition of surfactants, and or enzymes.

The extended release suspension composition of the present invention provides the consistent *in-vivo* release which ensures steady and predictable active ingredient release with minimal inter and intra subject variation throughout the shelf life of the composition.

The term "substantial," as used herein refers to any value which lies within the range as defined by a variation of up to ±15 from the average value.

The extended release suspension composition of the present invention may be in the form of a suspension or a reconstituted powder for suspension. This powder for suspension may be reconstituted with a pharmaceutically acceptable vehicle or a suspension base to form an extended release suspension composition.

The term "suspension base," as used herein, refers to a medium which is used to suspend the coated cores of the active ingredient or to reconstitute the extended release powder for suspension of the active ingredient. The suspension base comprises a pharmaceutically acceptable vehicle, one or more osmogents, and pharmaceutically acceptable excipients.

The pharmaceutically acceptable vehicle as used herein means an aqueous vehicle.

The diameter of the coated cores of the present invention ranges from about 10 µm to about 2000 µm, particularly from about 50 µm to about 1000 µm, and more particularly from about 150 µm to about 500 µm. The finer sizes of the coated cores help in avoiding grittiness in the mouth and are therefore are more acceptable. The cores of the present invention may comprise one or more pharmaceutically acceptable excipients such as a binder, and optionally one more osmogents.

The active ingredient of the present invention includes at least metformin or guanfacine. The active ingredient of the present invention can be present in the form of a free base or in the form of pharmaceutically acceptable salts. Further, the extended release suspension compositions of the present invention permit ready dose titration, i.e., adjusting the dose of the active ingredient based on recommended dose range and frequency until the desired therapeutic effect is achieved. In particular, the active ingredients used in the present invention are active ingredients with a high dose.

The extended release suspension compositions of the present invention may further include an immediate release component of the active ingredient to have a biphasic or pulsatile type of release. The immediate release component may be present in the form of a powder, a pellet, a bead, a spheroid, or a granule. Alternatively, the immediate release component may be present in the form of an immediate release coating over the coated cores. The immediate release component may help in providing an immediate therapeutic effect which could be subsequently followed by an extended therapeutic effect over a longer duration of time. Depending upon the type of polymer and percentage weight gain of the coating, the lag between the two phases can be adjusted to get the desired release profile.

The extended release suspension composition of the present invention may comprise two or more different active ingredients with different type of release profiles. One of the active ingredients provides the extended release, whereas another active ingredient may provide the immediate release or the extended release.

The extended release suspension composition of the present invention may further comprise two or more incompatible active ingredients present in a single composition. One of the active ingredients would be present in the form of coated cores providing the extended release and another incompatible active ingredient may be present in the form of a powder, a pellet, a bead, a spheroid, or a granule providing the immediate release or the extended release.

The extended release suspension compositions of the present invention are homogenous and deliver the desired dose of the active ingredient in every use without any risk of overdosing or underdosing.

The extended release suspension composition of the present invention has a pH ranging from about 2 to about 10.

The release-controlling polymers used to form the extended release coating are selected from a group comprising a pH-dependent polymer, a pH-independent polymer, or mixtures thereof.

Suitable examples of pH-dependent polymers are selected from the group comprising acrylic copolymers such as methacrylic acid and methyl methacrylate copolymers, *e.g*., Eudragit^{®} L 100 and Eudragit^{®} S 100, methacrylic acid and ethyl acrylate copolymers, *e.g*., Eudragit^{®} L 100-55 and Eudragit^{®} L 30 D-55, dimethylaminoethyl methacrylate and butyl methacrylate and methyl methacrylate copolymers *e.g*., Eudragit^{®} E 100, Eudragit^{®} E PO, methyl acrylate and methacrylic acid and octyl acrylate copolymers, styrene and acrylic acid copolymers, butyl acrylate and styrene and acrylic acid copolymers, and ethylacrylate-methacrylic acid copolymer; cellulose acetate phthalate; cellulose acetate succinates; hydroxyalkyl cellulose phthalates such as hydroxypropylmethyl cellulose phthalate; hydroxyalkyl cellulose acetate succinates such as hydroxypropylmethyl cellulose acetate succinate; vinyl acetate phthalates; vinyl acetate succinate; cellulose acetate trimelliate; polyvinyl derivatives such as polyvinyl acetate phthalate, polyvinyl alcohol phthalate, polyvinyl butylate phthalate, and polyvinyl acetoacetal phthalate; zein; shellac; or mixtures thereof.

Suitable examples of pH-independent polymers are selected from the group comprising cellulosic polymers such as ethyl cellulose, methyl cellulose, hydroxyethyl cellulose, hydroxy-propyl cellulose, hydroxyethylmethyl cellulose, hydroxypropylmethyl cellulose, and carboxy methylcellulose; acrylic copolymers such as methacrylic acid copolymers, *e.g*., Eudragit^{®} RS, Eudragit^{®} RL, Eudragit^{®} NE 30 D; cellulose acetate; polyethylene derivatives *e.g*., polyethylene glycol and polyethylene oxide; polyvinyl alcohol; polyvinyl acetate; gums *e.g*., guar gum, locust bean gum, tragacanth, carrageenan, alginic acid, gum acacia, gum arabic, gellan gum, and xanthan gum; triglycerides; waxes, *e.g*., Compritol^{®}, Lubritab^{®}, and Gclucires^{®}: lipids; fatty acids or their salts/derivatives; a mixture of polyvinyl acetate and polyvinyl pyrrolidone, *e.g*., Kollidon^{®} SR; or mixtures thereof.

The term "osmogent," as used herein, refers to all pharmaceutically acceptable inert water-soluble compounds that can imbibe water and/or aqueous biological fluids. Suitable examples of osmogents or pharmaceutically acceptable inert water-soluble compounds are selected from the group comprising carbohydrates such as xylitol, mannitol, sorbitol, arabinose, ribose, xylose, glucose, fructose, mannose, galactose, sucrose, maltose, lactose, dextrose and raffinose; water-soluble salts of inorganic acids such as magnesium chloride, magnesium sulfate, potassium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, potassium dihydrogen phosphate, and sodium phosphate tribasic; water-soluble salts of organic acids such as sodium acetate, potassium acetate, magnesium succinate, sodium benzoate, sodium citrate, and sodium ascorbate; water-soluble amino acids such as glycine, leucine, alanine, methionine; urea or its derivatives; propylene glycol; glycerin; polyethylene oxide; xanthan gum; hydroxypropylmethyl cellulose; or mixtures thereof. Particularly, the osmogents used in the present invention are xylitol, mannitol, glucose, lactose, sucrose, and sodium chloride. The term "pharmaceutically acceptable excipients," as used herein, refers to excipients that are routinely used in pharmaceutical compositions. The pharmaceutically acceptable excipients may comprise glidants, sweeteners, suspending agents, anti-caking agents, wetting agents, preservatives, buffering agents, flavoring agents, anti-oxidants, chelating agents, or combinations thereof.

Suitable glidants are selected from the group comprising silica, calcium silicate, magnesium silicate, colloidal silicon dioxide, cornstarch, talc, stearic acid, magnesium stearate, calcium stearate, sodium stearyl fumarate, hydrogenated vegetable oil, or mixtures thereof.

Suitable sweeteners are selected from the group comprising saccharine or its salts such as sodium, potassium, or calcium, cyclamate or its salt, aspartame, alitame, acesulfame or its salt, stevioside, glycyrrhizin or its derivatives, sucralose, or mixtures thereof.

Suitable suspending agents are selected from the group comprising cellulose derivatives such as co-processed spray dried forms of microcrystalline cellulose and carboxymethyl cellulose sodium, hydroxypropyl cellulose, hydroxyethyl cellulose, hydroxypropylmethyl cellulose, methylcellulose, carboxymethyl cellulose and its salts/derivatives, and microcrystalline cellulose; carbomers; gums such as locust bean gum, xanthan gum, tragacanth gum, arabinogalactan gum, agar gum, gellan gum, guar gum, apricot gum, karaya gum, sterculia gum, acacia gum, gum arabic, and carrageenan; pectin; dextran; gelatin; polyethylene glycols; polyvinyl compounds such as polyvinyl acetate, polyvinyl alcohol, and polyvinyl pyrrolidone; sugar alcohols such as xylitol and mannitol; colloidal silica; or mixtures thereof. Co-processed spray dried forms of microcrystalline cellulose and carboxymethyl cellulose sodium have been marketed under the trade names Avicel^{®} RC-501, Avicel^{®} RC-581, Avicel^{®} RC-591, and Avicel^{®} CL-611.

Suitable anti-caking agents are selected from the group comprising colloidal silicon dioxide, tribasic calcium phosphate, powdered cellulose, magnesium trisilicate, starch, or mixtures thereof.

Suitable wetting agents are selected from the group comprising anionic, cationic, nonionic, or zwitterionic surfactants, or combinations thereof. Suitable examples of wetting agents are sodium lauryl sulphate; cetrimide; polyethylene glycols; polyoxyethylene-polyoxypropylene block copolymers such as poloxamers; polyglycerin fatty acid esters such as decaglyceryl monolaurate and decaglyceryl monomyristate; sorbitan fatty acid esters such as sorbitan monostearate; polyoxyethylene sorbitan fatty acid esters such as polyoxyethylene sorbitan monooleate; polyethylene glycol fatty acid esters such as polyoxyethylene monostearate; polyoxyethylene alkyl ethers such as polyoxyethylene lauryl ether; polyoxyethylene castor oil; or mixtures thereof.

Suitable preservatives are selected from the group comprising parabens such as methyl paraben and propyl paraben; sodium benzoate; or mixtures thereof.

Suitable buffering agents are selected from the group comprising citric acid, sodium citrate, sodium phosphate, potassium citrate, acetate buffer, or mixtures thereof.

Suitable flavoring agents are selected from the group consisting of peppermint, grapefruit, orange, lime, lemon, mandarin, pineapple, strawberry, raspberry, mango, passion fruit, kiwi, apple, pear, peach, apricot, cherry, grape, banana, cranberry, blueberry, black currant, red currant, gooseberry, lingon berries, cumin, thyme, basil, camille, valerian, fennel, parsley, chamomile, tarragon, lavender, dill, bargamot, salvia, aloe vera balsam, spearmint, eucalyptus, or combinations thereof.

Suitable anti-oxidants are selected from the group comprising butylated hydroxytoluene (BHT), butylated hydroxyanisole (BHA), sodium metabisulfite, ascorbic acid, propyl gallate, thiourea, tocopherols, beta-carotene, or mixtures thereof.

Suitable chelating agents are selected from the group comprising ethylenediamine tetraacetic acid or derivatives/salts thereof, *e.g*., disodium edetate; dihydroxyethyl glycine; glucamine; acids, *e.g*., citric acid, tartaric acid, gluconic acid, and phosphoric acid; or mixtures thereof.

Suitable binders are selected from the group comprising polyvinyl pyrrolidone, starch, pregelatinized starch, hydroxypropylmethyl cellulose, hydroxyethyl cellulose, methyl cellulose, sodium carboxymethyl cellulose, gums, acrylate polymers, or mixtures thereof.

The cores of the present invention comprising the active ingredient can be prepared by any method known in the art, *e.g*., extrusion-spheronoization, wet granulation, dry granulation, hot-melt extrusion granulation, spray drying, and spray congealing. Alternatively, the active ingredient can be layered onto an inert particle to form the core.

Further, the active ingredient particles can be directly coated with a release-controlling polymer to form the microparticles or microcapsules. The microparticles or microcapsules can be prepared by a process of homogenization, solvent evaporation, coacervation phase separation, spray drying, spray congealing, polymer precipitation, or supercritical fluid extraction.

The extended release suspension compositions of the present invention may further comprise one or more seal coating layers which may be applied before and/or after the functional coating layer. The seal coating layer may comprise of one or more film forming polymers and coating additives.

Examples of film-forming polymers include ethylcellulose, hydroxypropyl methylcellulose, hydroxypropylcellulose, methylcellulose, carboxymethyl cellulose, hydroxymethylcellulose, hydroxyethylcellulose, cellulose acetate, hydroxypropyl methylcellulose phthalate, cellulose acetate phthalate, cellulose acetate trimellitate; waxes such as polyethylene glycol; methacrylic acid polymers such as Eudragit^{®}. Alternatively, commercially available coating compositions comprising film-forming polymers marketed under various trade names, such as Opadry^{®} may also be used.

The coating additives used in the present invention are selected from the group comprising plasticizers, opacifiers, anti-tacking agents, coloring agents, or combinations thereof.

Suitable plasticizers are selected from the group comprising triethyl citrate, dibutylsebacate, triacetin, acetylated triacetin, tributyl citrate, glyceryl tributyrate, diacetylated monoglyceride, rapeseed oil, olive oil, sesame oil, acetyl tributyl citrate, acetyl triethyl citrate, glycerin, sorbitol, diethyl oxalate, diethyl phthalate, diethyl malate, diethyl fumarate, dibutyl succinate, diethyl malonate, dioctyl phthalate, or combinations thereof.

Suitable opacifiers are selected from the group comprising titanium dioxide, manganese dioxide, iron oxide, silicon dioxide, or combinations thereof.

Suitable anti-tacking agents are selected from the group comprising talc, magnesium stearate, calcium stearate, stearic acid, silica, glyceryl monostearate, or combinations thereof.

Suitable coloring agents are selected from the group consisting of FD&C (Federal Food, Drug and Cosmetic Act) approved coloring agents; natural coloring agents; natural juice concentrates; pigments such as iron oxide, titanium dioxide, and zinc oxide; or combinations thereof.

Coating may be performed by applying the coating composition as a solution/suspension/blend using any conventional coating technique known in the art such as spray coating in a conventional coating pan, fluidized bed processor, dip coating, or compression coating. The percentage of the coating build-up shall be varied depending on the required extended release.

Suitable solvents used for granulation or for forming a solution or dispersion for coating are selected from the group consisting of water, ethanol, methylene chloride, isopropyl alcohol, acetone, methanol, or combinations thereof.

The extended release suspension compositions of the present invention may be packaged in a suitable package such as a bottle. The powder for suspension may be packaged in a suitable package such as a bottle or a sachet. Further, the sachet can be filled as a unit dose or a multidose sachet. The present invention further includes a co-package or a kit comprising two components, wherein one package or one component comprises a powder for suspension and another package or another component comprises a suspension base or a pharmaceutically acceptable vehicle.

The invention may be further illustrated by the following examples, which are for illustrative purposes only and should not be construed as limiting the scope of the invention in any way.

### EXAMPLES

### Example 1

| **Ingredients** | **Quantity (mg/mL)** |
|---|---|
| **Core** | |
| Metformin hydrochloride | 100.00 |
| Microcrystalline cellulose spheres | 90.00 |
| Hydroxypropylmethyl cellulose | 5.00 |
| Purified water | q.s. |

| **Extended Release Coating** | |
|---|---|
| Ethyl cellulose | 61.42 |
| Dibutyl sebacate | 6.82 |
| Acetone | q.s. |
| Purified water | q.s. |
| **Total Weight of Extended Release Beads** | **263.24 mg** |
| Xylitol | 450.00 |
| Xanthan gum | 1.500 |
| Microcrystalline cellulose - sodium carboxymethyl cellulose (Avicel^{®} CL61 1) | 20.00 |
| Strawberry flavor | 1.50 |

| **Vehicle** | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Xylitol, xanthan gum, microcrystalline cellulose - sodium carboxymethyl cellulose, and strawberry flavor were mixed with the coated beads of step 4.
6. The mixture of step 5 was dispersed in required amount of purified water to obtain the extended release suspension composition.

### In-Vitro Studies

*In-vitro* release of metformin from the extended release suspension composition prepared as per Example 1 was determined by the dissolution for metformin using USP type II apparatus at 100 rpm, in 1000 mL of phosphate buffer with pH 6.8 at 37°C. The results of the release studies are represented in Table 1.

**Table 1: Percentage (%) of the In-Vitro Metformin Release in USP Type II Apparatus (Media: Phosphate Buffer, pH 6.8.1000 mL, and 100 rpm)**

| **Number of Days** | **0** | **15** | **30** |
|---|---|---|---|
| **Time (hours)** | **Percentage of Metformin Release** | | |
| 2 | 9 | 9 | 10 |
| 3 | 34 | 35 | 37 |
| 4 | 56 | 57 | 57 |
| 5 | 69 | 69 | 68 |
| 6 | 76 | 76 | 75 |
| 8 | 85 | 85 | 84 |
| 10 | 90 | 92 | 89 |
| 12 | 93 | 94 | 92 |

From the above data, it is clear that the extended release suspension composition prepared according to Example 1 provides substantially similar *in-vitro* metformin release for 30 days.

### Example 2

| **Ingredients** | **Quantity (mg/mL)** |
|---|---|
| **Core** | |
| Metformin hydrochloride | 100.00 |
| Microcrystalline cellulose spheres | 90.00 |
| Hydroxypropylmethyl cellulose | 5.00 |
| Purified water | q.s. |

| **Extended Release Coating** | |
|---|---|
| Ethyl cellulose | 61.42 |
| Dibutyl sebacate | 6.82 |
| Acetone | q.s. |
| Purified water | q.s. |
| **Total Weight of Extended Release Beads** | **263.24 mg** |
| Xylitol | 450.00 |
| Xanthan gum | 1.50 |
| Microcrystalline cellulose - sodium carboxymethyl cellulose (Avicel^{®} CL61 1) | 20.00 |
| Strawberry flavor | 1.50 |

| **Vehicle** | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Xylitol, xanthan gum, microcrystalline cellulose - sodium carboxymethyl cellulose, and strawberry flavor were mixed with the coated beads of step 4 to obtain a powder for suspension.
6. The powder for suspension as per step 5 is reconstituted with required amount of purified water when required to obtain the extended release suspension composition.

### Example 3

| **Ingredients** | **Quantity (mg/5 mL)** |
|---|---|
| **Core** | |
| Metformin hydrochloride | 500.00 |
| Microcrystalline cellulose spheres | 375.00 |
| Hydroxypropylmethyl cellulose | 25.00 |
| Purified water | q.s. |

| **Extended Release Coating** | |
|---|---|
| Ethyl cellulose | 340.20 |
| Dibutyl sebacate | 37.80 |
| Acetone | q.s. |
| Purified water | q.s. |
| **Total Weight of Extended Release Beads** | **1278.00 mg** |
| Xylitol | 2250.00 |
| Xanthan gum | 7.50 |
| Microcrystalline cellulose - sodium carboxymethyl cellulose (Avicel^{®} RC 591) | 100.00 |
| Strawberry flavor | 7.50 |

| **Vehicle** | |
|---|---|
| Purified water | q.s. to 5 mL |

### Procedure:

1. Metformin hydrochloride, microcrystalline cellulose, and hydroxypropylmethyl cellulose were sifted and mixed to obtain a blend.
2. The blend of step 1 was mixed with purified water to obtain a wet mass.
3. The wet mass of step 2 was extruded through an extruder.
4. The extrudates of step 3 were spherionized through a spherionizer to obtain beads.
5. The beads of step 4 were dried.
6. Ethyl cellulose and dibutyl sebacate were dispersed in a mixture of acetone and purified water.
7. The dried beads of step 5 were coated with the coating dispersion of step 6 to obtain a powder for suspension.
8. Xyltiol, xanthan gum, microcrystalline cellulose - sodium carboxymethyl cellulose, and strawberry flavor were dispersed in purified water to obtain the vehicle.
9. The powder for suspension of step 7 is reconstituted with the vehicle of step 8 when required to obtain the extended release suspension composition.

### Example 4

| **Ingredients** | **Quantity (mg/mL)** |
|---|---|
| **Core** | |
| Metformin hydrochloride | 80.00 |
| Microcrystalline cellulose spheres | 56.00 |
| Hydroxypropylmethyl cellulose | 4.00 |
| Purified water | q.s. |

| **Extended Release Coating** | |
|---|---|
| Ethyl cellulose | 45.00 |
| Dibutyl sebacate | 1.50 |
| Acetone | q.s. |
| Purified water | q.s. |
| **Total Weight of Extended Release Beads** | **186.50 mg** |
| Metformin hydrochloride | 20.00 |
| Xylitol | 450.00 |
| Microcrystalline cellulose - sodium carboxymethyl cellulose (Avicel^{®} CL-611) | 20.00 |
| Xanthan gum | 1.50 |
| Strawberry flavor | 2.00 |
| Sucralose | 0.50 |
| Sodium benzoate | 3.00 |
| Colloidal silicon dioxide | 3.50 |

| **Vehicle** | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Metformin hydrochloride, xylitol, microcrystalline cellulose - sodium carboxymethyl cellulose, xanthan gum, strawberry flavor, sucralose, sodium benzoate, and colloidal silicon dioxide were mixed.
6. The coated beads of step 4 were mixed with the mixture of step 5 to obtain a powder for suspension.
7. The powder for suspension of step 6 is reconstituted with required amount of purified water when required to form the extended release suspension composition.

### In-Vitro Studies

The extended release suspension composition prepared according to Example 4 was stored at room temperature for 66 days. This extended release suspension was analyzed for the *in-vitro* dissolution at 0, 30, and 66 days using USP type II apparatus at 100 rpm, in 1000 mL of phosphate buffer with pH 6.8 at 37°C. The results of the release studies are represented in Table 2.

**Table 2: Percentage (%) of the In-Vitro Metformin Release in USP Type II Apparatus (Media: Phosphate Buffer, pH 6.8.1000 mL, and 100 rpm)**

| **Number of Days** | **0** | **30** | **66** |
|---|---|---|---|
| **Time (hours)** | **Percentage of Metformin Release** | | |
| 0.5 | 27.0 | 25.6 | 26.3 |
| 1 | 30.9 | 31.5 | 31.6 |
| 2 | 56.9 | 58.3 | 50.9 |
| 3 | 74.9 | 72.8 | 70.6 |
| 4 | 85.6 | 81.7 | 81.6 |
| 5 | 89.1 | 87.7 | 87.4 |
| 6 | 94.9 | 90.3 | 92.3 |
| 8 | 97.7 | 93.5 | - |
| 10 | 99.4 | 95.3 | - |
| 12 | 103.4 | 99.4 | 1000 |

From the above data, it is clear that the extended release suspension composition prepared according to Example 4 provides substantially similar *in-vitro* metformin release for 66 days.

The powder for suspension prepared as per Example 4 (till step 6) was kept for one month at accelerated conditions *i.e.,* 40°C/75% R.H. After one month, the powder for suspension was reconstituted with required amount of purified water and this extended release suspension composition was kept for 66 days at room temperature. The *in-vitro* dissolution was determined at 0, 36, and 66 days using USP type II apparatus at 100 rpm, in 1000 mL of phosphate buffer with pH 6.8 at 37°C. The results of the release studies are represented in Table 3.

**Table 3: Percentage (%) of the In-Vitro Metformin Release in USP Type II Apparatus (Media: Phosphate Buffer, pH 6.8.1000 mL, and 100 rpm)**

| **Number of Days After Reconstitution** | **0** | **36** | **66** |
|---|---|---|---|
| **Time (hours)** | **Percentage of Metformin Release** | | |
| 0.5 | 28.8 | 26.2 | 27.0 |
| 1 | 32.4 | 33 | 32.0 |
| 2 | 57.6 | 50.5 | 53.0 |
| 3 | 74.8 | 70.3 | 67.0 |
| 4 | 83.1 | 80.7 | 83.0 |
| 5 | 89.2 | 85.9 | 87.0 |
| 6 | 91.3 | 91.2 | 92.0 |
| 8 | 95.2 | - | 95.0 |
| 10 | 96.6 | - | 97.0 |
| 12 | 98.6 | 101.3 | 100.0 |

From the above data, it is clear that the extended release powder prepared according to Example 4 stored at accelerated conditions for one month, upon reconstitution and storage for 66 days at room temperature provides substantially similar *in-vitro* metformin release for 66 days. The results are shown in Figure 1.

### Example 5

| **Ingredients** | **Quantity (mg/mL)** |
|---|---|
| Core | |
| Metformin hydrochloride | 80.00 |
| Microcrystalline cellulose spheres | 56.00 |
| Hydroxypropylmethyl cellulose | 4.00 |
| Purified water | q.s. |

| **Extended Release Coating** | |
|---|---|
| Ethyl cellulose | 50.40 |
| Dibutyl sebacate | 5.60 |
| Acetone | q.s. |
| Purified water | q.s. |
| **Total Weight of Extended Release Beads** | **196.00 mg** |
| Metformin hydrochloride | 20.00 |
| Xylitol | 450.00 |
| Microcrystalline cellulose - sodium carboxymethyl cellulose (Avicel^{®} CL-611) | 20.00 |
| Xanthan gum | 1.50 |
| Strawberry flavor | 2.00 |
| Sucralose | 0.50 |

| **Vehicle** | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Metformin hydrochloride, xylitol, microcrystalline cellulose - sodium carboxymethyl cellulose, xanthan gum, strawberry flavor, and sucralose were mixed.
6. The coated beads of step 4 were mixed with the mixture of step 5 to form a powder for suspension.
7. The powder for suspension of step 6 is reconstituted with required amount of purified water when required to form the extended release suspension composition.

### In-Vitro Studies

The extended release suspension composition prepared as per Example 5 was stored at room temperature for 30 days. The *in-vitro* dissolution was determined at 0 and 30 days using USP type II apparatus at 100 rpm, in 1000 mL of phosphate buffer with pH 6.8 at 37°C. The results of the release studies are represented in Table 4.

**Table 4: Percentage (%) of the In-Vitro Metformin Release in USP Type II Apparatus (Media: Phosphate Buffer, pH 6.8, 1000 mL, and 100 rpm)**

| **Number of Days** | **0** | **30** |
|---|---|---|
| **Time (hours)** | **Percentage of Metformin Release** | |
| 0.5 | 22 | 24 |
| 1 | 31 | 34 |
| 2 | 58 | 61 |
| 4 | 83 | 89 |
| 5 | 86 | 93 |
| 6 | 91 | 96 |
| 8 | 95 | 101 |
| 10 | 97 | 102 |
| 12 | 99 | 103 |

From the above data, it is clear that the extended release suspension composition prepared according to Example 5 provides substantially similar *in-vitro* metformin release for 30 days.

The powder for suspension prepared as per Example 5 (till step 6) was kept for three months at accelerated conditions *i.e.,* 40°C/75% R.H. After three months, the powder for suspension was reconstituted with required amount of purified water and this extended release suspensions composition was kept for 32 days at room temperature. The *in-vitro* dissolution was determined at 0 and 32 days using USP type II apparatus at 100 rpm, in 1000 mL of phosphate buffer with pH 6.8 at 37°C. The results of the release studies are represented in Table 5.

**Table 5: Percentage (%) of the In-Vitro Metformin Release in USP Type II Apparatus (Media: Phosphate Buffer, pH 6.8, 1000 mL, and 100 rpm)**

| **Number of Days After Reconstitution** | **0** | **32** |
|---|---|---|
| **Time (hours)** | **Percentage of Metformin Release** | |
| 0.5 | 22 | 26 |
| 1 | 33 | 37 |
| 2 | 60 | 66 |
| 4 | 85 | 90 |
| 5 | 89 | 94 |
| 6 | 92 | 97 |
| 8 | 96 | 101 |
| 10 | 98 | 103 |
| 12 | 101 | 103 |

The powder for suspension prepared as per Example 5 (till step 6) was kept for six months at accelerated conditions *i.e.,* 40°C/75% R.H. After six months, the powder for suspension was reconstituted with required amount of purified water and this extended release suspensions composition was kept for 32 days at room temperature. The *in-vitro* dissolution was determined at 0 and 32 days using USP type II apparatus at 100 rpm, in 1000 mL of phosphate buffer with pH 6.8 at 37°C. The results of the release studies are represented in Table 6.

**Table 6: Percentage (%) of the In-Vitro Metformin Release in USP Type II Apparatus (Media: Phosphate Buffer, pH 6.8.1000 mL, and 100 rpm)**

| **Number of Days After Reconstitution** | **0** | **32** |
|---|---|---|
| **Time (hours)** | **Percentage of Metformin Release** | |
| 0.5 | 24 | 25 |
| 1 | 35 | 34 |
| 2 | 63 | 60 |
| 4 | 87 | 86 |
| 5 | 91 | 91 |
| 6 | 94 | 94 |
| 8 | 97 | 98 |
| 10 | 99 | 101 |
| 12 | 99 | 101 |

From the above data, it is clear that the extended release powder prepared according to Example 5 stored at accelerated conditions for three or six months, upon reconstitution and storage for 32 days at room temperature provides substantially similar *in-vitro* metformin release for 32 days. The results are presented in Figure 2.

### Example 6

| **Ingredients** | **Quantity (mg/mL)** |
|---|---|
| **Core** | |
| Metformin hydrochloride | 80.00 |
| Microcrystalline cellulose spheres | 56.00 |
| Hydroxypropylmethyl cellulose | 4.00 |
| Purified water | q.s. |

| **Extended Release Coating** | |
|---|---|
| Ethyl cellulose | 61.48 |
| Dibutyl sebacate | 1.52 |
| Acetone | q.s. |
| Purified water | q.s. |
| **Total Weight of Extended Release Beads** | **203.00 mg** |
| Metformin hydrochloride | 20.00 |
| Xylitol | 450.00 |
| Microcrystalline cellulose - sodium carboxymethyl cellulose (Avicel^{®} CL-611) | 20.00 |
| Xanthan gum | 1.50 |
| Strawberry flavor | 2.00 |
| Sucralose | 0.50 |
| Colloidal silicon dioxide | 3.50 |

| **Vehicle** | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Metformin hydrochloride, xylitol, microcrystalline cellulose - sodium carboxymethyl cellulose, xanthan gum, strawberry flavor, sucralose, and colloidal silicon dioxide were mixed.
6. The coated beads of step 4 were mixed with the mixture of step 5 to form a powder for suspension.
7. The powder for suspension of step 6 is reconstituted with required amount of purified water when required to form the extended release suspension composition.

### In-Vitro Studies

The extended release suspension composition prepared as per Example 6 was stored at room temperature for 30 days. The *in-vitro* dissolution was determined at 0 and 30 days using USP type II apparatus at 100 rpm, in 1000 mL of phosphate buffer with pH 6.8 at 37°C. The results of the release studies are represented in Table 7.

**Table 7: Percentage (%) of the In-Vitro Metformin Release in USP Type II Apparatus (Media: Phosphate Buffer, pH 6.8, 1000 mL, and 100 rpm)**

| **Number of Days** | **0** | **30** |
|---|---|---|
| **Time (hours)** | **Percentage of Metformin Release** | |
| 0.5 | 20 | 22 |
| 1 | 27 | 28 |
| 2 | 59 | 64 |
| 3 | 77 | 80 |
| 4 | 84 | 89 |
| 5 | 88 | 93 |
| 6 | 92 | 95 |
| 8 | 95 | 99 |
| 10 | 97 | 101 |
| 12 | 98 | 103 |

From the above *in-vitro* release data, it is evident that the extended release suspension composition prepared according to Example 6 provides the substantially similar *in-vitro* metformin release for 30 days.

The powder for suspension prepared as per Example 6 (till step 6) was kept for one month at accelerated conditions *i.e.,* 40°C/75% R.H. After one month, the powder for suspension was reconstituted with required amount of purified water and this extended release suspension composition was kept for 30 days at room temperature. The *in-vitro* dissolution was determined at 0 and 30 days using USP type II apparatus at 100 rpm, in 1000 mL of phosphate buffer with pH 6.8 at 37°C. The results of the release studies are represented in Table 8.

**Table 8: Percentage (%) of the In-Vitro Metformin Release in USP Type II Apparatus (Media: Phosphate Buffer, pH 6.8, 1000 mL, and 100 rpm)**

| **Number of Days After Reconstitution** | **0** | **30** |
|---|---|---|
| **Time (hours)** | **Percentage of Metformin Release** | |
| 0.5 | 20 | 19 |
| 1 | 26 | 26 |
| 2 | 57 | 57 |
| 3 | 74 | 74 |
| 4 | 82 | 80 |
| 5 | 86 | 85 |
| 6 | 90 | 88 |
| 8 | 92 | 91 |
| 10 | 94 | 93 |
| 12 | 96 | 94 |

From the above data, it is clear that the extended release powder prepared according to Example 6 stored at accelerated condition for one month, upon reconstitution and storage for 30 days at room temperature provides substantially similar *in-vitro* metformin release for 30 days. The results are presented in Figure 3.

### Osmolality Measurement of the Extended Release Suspension

The metformin extended release powder prepared according to the Example 6 (till step 6) was reconstituted with required amount of purified water. This suspension was shaken manually for at least 20 minutes. This suspension was then filtered and diluted with purified water and the osmolality was measured using Osmomat 030-D.

The osmolality of the suspension base was found to be 4.112 osmol/kg of the suspension base on day 0.

The osmolality of the suspension base was found to be 4.328 osmol/kg of the suspension base on day 7.

it is evident from the above data that the osmolality of the suspension base of the extended release suspension composition as per Example 6 remains equivalent for seven days.

### Osmolality Measurement of the External Phase

The metformin hydrochloride, xylitol, microcrystalline cellulose - sodium carboxymethyl cellulose, xanthan gum, strawberry flavor, sucralose, and colloidal silicon dioxide were mixed as per step 5 of Example 6. This mixture was reconstituted with required amount of purified water. This suspension was then filtered and diluted with purified water, and the osmolality was measured using Osmomat 030-D.

The osmolality of the suspension base *i.e.,* external phase was found to be 4.204 osmol/kg of the suspension base.

### Osmolality Measurement of the Internal Phase

Various solutions having various concentrations of osmogent (sodium chloride) were prepared as per Examples 6A-6F. The osmolalities of these solutions were measured using Osmomat 030-D.

| **Ingredient** | | **Example 6A** | **Example 6B** | **Example 6C** | **Example 6D** | **Example 6E** | **Example 6F** |
|---|---|---|---|---|---|---|---|
| | Sodium Chloride (mg) | 30.00 | 60.00 | 120.00 | 180.00 | 240.00 | 300.00 |
| | Purified water | q.s. to 1 mL | q.s. to 1 mL | q.s. to 1 mL | q.s. to 7.5 mL | q.s. to 1 mL | q.s. to 1 mL |
| | Osmolality (osmol/kg) | 0.910 | 1.787 | 3.574* | 5.361* | 7.148* | 8.935* |
| * Extrapolated using values of dilute solutions | | | | | | | |

The coated beads of step 4 were dispersed in different solutions as per Examples 6A-6F. These solutions were kept for seven days at room temperature. After seven days, each solution was analyzed by HPLC for metformin content. The results are represented in following Table 9.

**Table 9: Effect of Osmolality on Metformin Leaching**

| **Example** | **Osmolality (osmol/kg) of the solution** | **Metformin Content (%)** |
|---|---|---|
| 6A | 0.910 | 67.3 |
| 6B | 1.787 | 30.3 |
| 6C | 3.574* | 2.9 |
| 6D | 5.361* | 1.8 |
| 6E | 7.148* | 1.7 |
| 6F | 8.935* | 1.0 |
| * Extrapolated using values of dilute solutions | | |

From the above data, it is evident that the leaching of metformin from the coated beads into the solution was decreasing as the osmolality of the solution was increasing from Examples 6A-6F. The leaching is found to be significantly reduced from Example 6C onwards. The osmolality of Example 6C *i.e.,* 3.574 is considered as osmolality of the internal phase.

### Osmolality Ratio 1.176

### Dose Uniformity Data

The extended release suspension equivalent to 100 mL was prepared according to formula given in Example 6. This suspension was shaken manually for at least 20 minutes and then ten 7.5 mL samples were taken with a graduated syringe. The metformin content of each sample is determined by HPLC method [Inertsil ODS column (250 x 4.6 mm, 5 µm); mobile phase-buffer (pH 3.5):acetonitrile (95:5 v/v); flow rate of 1.5 mL/min; UV detection at 233 nm] The results are shown in Table 10.

**Table 10: Metformin Content (%w/w) For Each 7.5 mL of Suspension**

| **Sample Number** | **Metformin content (%) for each 7.5 mL of suspension** |
|---|---|
| 1 | 98.6 |
| 2 | 97.9 |
| 3 | 96.6 |
| 4 | 97.2 |
| 5 | 99.7 |
| 6 | 96.4 |
| 7 | 95.9 |
| 8 | 97.3 |
| 9 | 98.8 |
| 10 | 96.9 |
| Mean value | 97.5 |

From the above data, it is evident that the extended release suspension composition prepared as per Example 6 is homogeneous.

### Assay Data

The assay for the extended release suspension composition prepared as per Example 6 was determined at 0 day and after storage at room temperature for 30 days. The powder for suspension prepared as per Example 6 (till step 6) was kept for one month at 40°C/75% R.H. After one month, the powder for suspension was reconstituted with required amount of purified water and then assay was determined at 0 day and after storage at room temperature for 30 days.

The assay of metformin was determined by HPLC method [Inertsil ODS column (250 x 4.6 mm, 5 µm); mobile phase-buffer (pH 3.5):acetonitrile (95:5 v/v); flow rate of 1.5 mL/min; UV detection at 233 nm]. The results are shown in Table 11.

**Table 11: Assay for Metformin**

| **Condition** | **Assay (%) for metformin** | |
|---|---|---|
| | 0 day | 30 days |
| Initial | 97.0 | 99.5 |
| 1 month (40°C/75% R.H) | 97.4 | 98.9 |

It is evident from the above data that the extended release suspension composition prepared as per Example 6 is stable.

### Example 7

### Preparation of Extended Release Beads

| **Ingredients** | **Quantity (mg)** |
|---|---|
| **Core** | |
| Guanfacine hydrochloride | 1.15 |
| Microcrystalline cellulose spheres | 4.00 |
| Hydroxypropylmethyl cellulose | 30.00 |
| Mannitol | 10.00 |
| Purified Water | q.s. |

| **Extended Release Coating** | |
|---|---|
| Ethyl cellulose | 14.22 |
| Dibutyl sebacate | 1.58 |
| Acetone | q.s. |
| Purified Water | q.s. |
| **Total Weight of Extended Release Beads** | **60.95 mg** |

### Procedure:

1. Guanfacine hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.

Various solutions having various concentrations of osmogent (sodium chloride) were prepared as per Examples 7A-7D. The osmolalities of these solutions were measured using Osmomat 030-D.

| **Ingredient** | **Example 7A** | **Example 7B** | **Example 7C** | **Example 7D** |
|---|---|---|---|---|
| Sodium Chloride (mg) | 30.00 | 60.00 | 120.00 | 180.00 |
| Purified water | q.s. to 1 mL | q.s. to 1 mL | q.s. to 1 mL | q.s. to 1 mL |
| Osmolality (osmol/kg) | 0.910 | 1.787 | 3.574* | 5.361* |
| * Extrapolated using values of dilute solutions | | | | |

Sodium chloride was dissolved in purified water as per Examples7A-7D. The osmolality of these solutions were measured using Osmomat 030-D.

The coated beads of step 4 were dispersed in different suspension bases as per Examples7A-7D. These suspensions were kept for seven days at room temperature. After seven days, each suspension was filtered and diluted with purified water. These were then analyzed by using HPLC for guanfacine content. The results are represented in following Table 12.

**Table 12: Effect of Osmolality on Guanfacine Leaching**

| **Example** | **Osmolality (osmol/kg) of the solution** | **Guanfacine Content (%)** |
|---|---|---|
| 7A | 0.910 | 69.80 |
| 7B | 1.787 | 8.90 |
| 7C | 3.574* | 1.30 |
| 7D | 5.361* | 0.30 |
| *Extrapolated using values of dilute solutions | | |

From the above data, it is evident that the leaching of guanfacine from the coated beads into the solution was decreasing as the osmolality of the solution was increasing from Examples 7A-7D.

### Example 8

The following example is not according to the claims of the invention:

| **Ingredients** | **Quantity (mg/mL)** |
|---|---|
| **Core** | |
| Valacyclovir hydrochloride (equivalent to 100 mg of valacyclovir) | 111.24 |
| Microcrystalline cellulose spheres | 70.00 |
| Hydroxypropylmethyl cellulose | 5.56 |
| Purified water | q.s. |

| **Extended Release Coating** | |
|---|---|
| Ethyl cellulose | 45.58 |
| Dibutyl sebacate | 1.13 |
| Acetone | q.s. |
| Purified water | q.s. |
| **Total Weight of Extended Release Beads** | **233.51 mg** |
| Xylitol | 450.00 |
| Xanthan gum | 1.50 |
| Microcrystalline cellulose - sodium carboxymethyl cellulose (Avicel^{®} CL 611) | 20.00 |
| Strawberry flavor | 1.50 |

| **Vehicle** | |
|---|---|
| Purified water | q.s to 1.0 ml |

### Procedure:

1. Valacyclovir hydrochloride and hydroxypropylmethyl cellulose are dissolved in purified water.
2. Microcrystalline cellulose spheres are coated with the solution of step 1.
3. Ethyl cellulose and dibutyl sebacate are dispersed in a mixture of acetone and purified water.
4. The beads of step 2 are coated with the coating dispersion of step 3.
5. Xylitol, xanthan gum, microcrystalline cellulose - sodium carboxymethyl cellulose, strawberry flavor are mixed.
6. The coated beads of step 4 are mixed with the mixture of step 5 to form a powder for suspension.
7. The powder for suspension of step 6 is reconstituted with required amount of purified water when required to form the extended release suspension composition.

### Example 9

The following example is not according to the claims of the invention:

| **Ingredients** | **Quantity (mg/5mL)** |
|---|---|
| **Core** | |
| Amoxicillin | 1000.00 |
| Microcrystalline cellulose spheres | 200.00 |
| Polyvinylpyrrolidone | 60.00 |
| Purified water | q.s. |

| **Extended Release Coating** | |
|---|---|
| Ethyl cellulose | 500.00 |
| Dibutyl sebacate | 50.00 |
| Acetone | q.s. |
| Purified water | q.s. |
| **Total Weight of Extended Release Beads** | **1810.00 mg** |
| Clavulanic acid | 62.50 |
| Lemon flavor | 1.50 |
| Xylitol | 450.00 |
| Microcrystalline cellulose - sodium carboxymethyl cellulose (Avicel^{®} CL 611) | 20.00 |
| Strawberry flavor | 1.50 |

| **Vehicle** | |
|---|---|
| Purified water | q.s to 5.0 mL |

### Procedure:

1. Amoxicillin and polyvinylpyrrolidone are dispersed in purified water.
2. Microcrystalline cellulose spheres are coated with the solution of step 1.
3. Ethyl cellulose and dibutyl sebacate are dispersed in a mixture of acetone and purified water.
4. The beads of step 2 are coated with the coating dispersion of step 3.
5. Clavulanic acid, lemon flavor, xylitol, microcrystalline cellulose-sodium carboxymethyl cellulose, strawberry flavor are mixed.
6. The coated beads of step 4 are mixed with the mixture of step 5 to form a powder for suspension.
7. The powder for suspension of step 6 is reconstituted with required amount of purified water when required to form the extended release suspension composition.

### Example 10

The following example is not according to the claims of the invention:

| **Ingredients** | **Quantity (mg/mL)** |
|---|---|
| **Core** | |
| Esomeprazole magnesium | 44.50 |
| Non-pareil seeds | 100.00 |
| Hydroxypropyl cellulose | 20.00 |
| Crospovidone | 30.00 |
| Purified water | q.s. |

| **Seal Coating** | |
|---|---|
| Hydroxypropyl methyl cellulose | 14.98 |
| Polyethylene glycol | 1.49 |
| Talc | 2.98 |
| Purified water | q.s. |

| **Extended Release Coating** | |
|---|---|
| Methacrylic acid copolymer dispersion (Eudragit^{®} L30 D-55) | 33.47 |
| Polyethylene glycol | 3.35 |
| Talc | 12.72 |
| Titanium dioxide | 3.95 |
| Purified water | q.s. |
| Lubrication | |
| Talc | 12.56 |
| **Total Weight of Extended Release Beads** | **280.00 mg** |
| Xylitol | 450.00 |

| **Vehicle** | |
|---|---|
| Purified water | q.s to 1.0 mL |

### Procedure:

1. Esomeprazole magnesium, hydroxypropyl cellulose, crospovidone are dispersed in purified water and is stirred to get form a dispersion.
2. The non-pareil seeds are coated with dispersion of step 1.
3. The hydroxypropylmethyl cellulose, polyethylene glycol, and talc are dispersed in purified water to get a dispersion.
4. The coated pellets of step 2 are coated with the dispersion of step 3.
5. The polyethylene glycol, methacrylic acid copolymer dispersion, talc, and titanium dioxide are dispersed in purified water to get a dispersion.
6. The coated pellets of step 4 are coated with the dispersion of step 5.
7. The coated pellets of step 6 are lubricated with talc.
8. The lubricated pellets of step 7 are mixed with xylitol to obtain a powder for suspension.
9. The powder for suspension of step 8 is reconstituted with required amount of purified water when required to form the extended release suspension composition.

## Claims

1. A stable extended release suspension composition comprising:
(a) multiple coated cores comprising:
(i) a core comprising an active ingredient; and
(ii) a coating layer over said core comprising one or more release-controlling polymers;
(b) a suspension base comprising an osmogent, wherein the suspension base generates a hypertonic condition such that there is no substantial change in the *in-vitro* dissolution release profile of the extended release suspension compositions upon storage for at least seven days,
wherein the active ingredient is selected from the group comprising metformin or guanfacine.

2. The extended release suspension composition of claim 1, wherein the composition is a suspension or a reconstituted powder for suspension.

3. The extended release suspension composition of claim 1, wherein the active ingredient is layered onto an inert particle to form the core.

4. The extended release suspension composition of claim 3, wherein the inert particle is selected from the group comprising a non-pareil seed, a microcrystalline cellulose sphere, a dibasic calcium phosphate bead, a mannitol bead, a silica bead, a tartaric acid pellet, or a wax based pellet.

5. The extended release suspension composition of claim 1, wherein the osmogent is selected from the group comprising carbohydrates such as xylitol, mannitol, sorbitol, arabinose, ribose, xylose, glucose, fructose, mannose, galactose, sucrose, maltose, lactose, dextrose and raffinose; water-soluble salts of inorganic acids such as magnesium chloride, magnesium sulfate, potassium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, potassium dihydrogen phosphate, and sodium phosphate tribasic; water-soluble salts of organic acids such as sodium acetate, potassium acetate, magnesium succinate, sodium benzoate, sodium citrate, and sodium ascorbate; water-soluble amino acids such as glycine, leucine, alanine, methionine; urea or its derivatives; propylene glycol; glycerin; polyethylene oxide; xanthan gum; hydroxypropylmethyl cellulose; or mixtures thereof.

6. The extended release suspension composition of claim 1, wherein the release-controlling polymer is selected from the group comprising a pH-dependent polymer, a pH-independent polymer, or mixtures thereof.

7. The extended release suspension composition of claim 6, wherein the pH-dependent polymer is selected from the group comprising acrylic copolymers such as methacrylic acid and methyl methacrylate copolymers, *e.g*., Eudragit^{®} L 100 and Eudragit^{®} S 100, methacrylic acid and ethyl acrylate copolymers, *e.g.,* Eudragit^{®} L 100-55 and Eudragit^{®} L 30 D-55, dimethylaminoethyl methacrylate and butyl methacrylate and methyl methacrylate copolymer *e.g*., Eudragit^{®} E 100, Eudragit^{®} E PO, methyl acrylate and methacrylic acid and octyl acrylate copolymers, styrene and acrylic acid copolymers, butyl acrylate and styrene and acrylic acid copolymers, and ethylacrylate-methacrylic acid copolymer; cellulose acetate phthalate; cellulose acetate succinates; hydroxyalkyl cellulose phthalates such as hydroxypropylmethyl cellulose phthalate; hydroxyalkyl cellulose acetate succinates such as hydroxypropylmethyl cellulose acetate succinate; vinyl acetate phthalates; vinyl acetate succinate; cellulose acetate trimelliate; polyvinyl derivatives such as polyvinyl acetate phthalate, polyvinyl alcohol phthalate, polyvinyl butylate phthalate, and polyvinyl acetoacetal phthalate; zein; shellac; or mixtures thereof.

8. The extended release suspension composition of claim 6, wherein the pH-independent polymer is selected from the group comprising cellulosic polymers such as ethyl cellulose, methyl cellulose, hydroxyethyl cellulose, hydroxypropyl cellulose, hydroxyethylmethyl cellulose, hydroxypropylmethyl cellulose, and carboxy methylcellulose; acrylic copolymers such as methacrylic acid copolymers, *e.g*., Eudragit^{®} RS, Eudragit^{®} RL, Eudragit^{®} NE 30 D; cellulose acetate; polyethylene derivatives *e.g*., polyethylene glycol and polyethylene oxide; polyvinyl alcohol; polyvinyl acetate; gums *e.g*., guar gum, locust bean gum, tragacanth, carrageenan, alginic acid, gum acacia, gum arabic, gellan gum, and xanthan gum; triglycerides; waxes, *e.g*., Compritol^{®}, Lubritab^{®}, and Gelucires"'; lipids; fatty acids or their salts/derivatives; a mixture of polyvinyl acetate and polyvinyl pyrrolidone, *e.g*., Kollidon ^{®} SR; or mixtures thereof.

9. The extended release suspension composition of the claim 1, wherein the suspension base further comprises one or more pharmaceutically acceptable excipients selected from the group comprising suspending agents, anti-caking agents, wetting agents, preservatives, buffering agents, flavoring agents, anti-oxidants, and chelating agents.

## Patentansprüche

1. Stabile Suspensionszusammensetzung mit verlängerter Freisetzung, umfassend:
(a) mehrfach beschichtete Kerne, umfassend:
(i) einen Kern, der einen Wirkstoff umfasst; und
(ii) eine Beschichtungsschicht über dem Kern, umfassend ein oder mehrere freisetzungsregulierende Polymere;
(b) eine Suspensionsbasis, die ein Osmosemittel umfasst, wobei die Suspensionsbasis einen hypertonischen Zustand erzeugt, sodass keine wesentliche Veränderung in dem *In-*vitro-Auflösungsfreisetzungsprofil der Suspensionszusammensetzungen mit verlängerter Freisetzung bei Lagerung für mindestens sieben Tage erfolgt,
wobei der Wirkstoff aus der Gruppe ausgewählt ist, die Metformin oder Guanfacin umfasst.

2. Suspensionszusammensetzung mit verlängerter Freisetzung nach Anspruch 1, wobei die Zusammensetzung eine Suspension oder ein rekonstituiertes Pulver für die Suspension ist.

3. Suspensionszusammensetzung mit verlängerter Freisetzung nach Anspruch 1, wobei der Wirkstoff auf ein inertes Teilchen geschichtet ist, um den Kern zu bilden.

4. Suspensionszusammensetzung mit verlängerter Freisetzung nach Anspruch 3, wobei das inerte Teilchen aus der Gruppe ausgewählt ist, die einen Starterkern, eine mikrokristalline Zellulosekugel, ein zweibasisches Kalziumposphatkügelchen, ein Mannitolkügelchen, ein Silikakügelchen, ein Weinsäurepellet oder ein wachsbasiertes Pellet umfasst.

5. Suspensionszusammensetzung mit verlängerter Freisetzung nach Anspruch 1, wobei das Osmosemittel aus der Gruppe ausgewählt ist, die Kohlenhydrate wie Xylit, Mannit, Sorbit, Arabinose, Ribose, Xylose, Glucose, Fructose, Mannose, Galactose, Sucrose, Maltose, Lactose, Dextrose und Raffinose; wasserlösliche Salze anorganischer Säuren wie Magnesiumchlorid, Magnesiumsulfat, Kaliumsulfat, Lithiumchlorid, Natriumchlorid, Kaliumchlorid, Lithiumhydrogenphosphat, Natriumhydrogenphosphat, Kaliumhydrogenphosphat, Lithiumdihydrogenphosphat, Natriumdihydrogenphosphat, Kaliumdihydrogenphosphat und Natriumphosphat dreibasisch; wasserlösliche Salze organischer Säuren wie Natriumacetat, Kaliumacetat, Magnesiumsuccinat, Natriumbenzoat, Natriumcitrat und Natriumascorbat; wasserlösliche Aminosäuren wie Glycin, Leucin, Alanin, Methionin; Harnstoff oder dessen Derivate; Propylenglycol; Glycerin; Polyethylenoxid; Xanthangummi; Hydroxypropylmethylcellulose; oder Gemische davon umfasst.

6. Suspensionszusammensetzung mit verlängerter Freisetzung nach Anspruch 1, wobei das freisetzungsregulierende Polymer aus der Gruppe ausgewählt ist, die ein pH-Wert-abhängiges Polymer, ein pH-Wert-unabhängiges Polymer oder Gemische davon umfasst.

7. Suspensionszusammensetzung mit verlängerter Freisetzung nach Anspruch 6, wobei das pH-Wert-abhängige Polymer aus der Gruppe ausgewählt ist, die Acryl-Copolymere wie Methacrylsäure- und Methylmethacrylat-Copolymere, *z*. *B.* Eudragit^{®} L 100 und Eudragit^{®} S 100, Methacrylsäure- und Ethylacrylat-Copolymere, *z*. *B.* Eudragit^{®} L 100-55 und Eudragit^{®} L 30 D-55, Dimethylaminoethylmethacrylat- und Butylmethacrylat- und Methylmethacrylat-Copolymer, *z*. *B.* Eudragit^{®} E 100, Eudragit^{®} E PO, Mehthylacrylat- und Methacrylsäure- und Octylacrylat-Copolymere, Styren- und Acrylsäure-Copolymere, Butylacrylat- und Styren- und Acrylsäure-Copolymere und Ethylacrylatmethacrylsäure-Copolymer; Celluloseacetatphthalat; Cellulosecetatsuccinate; Hydroxyalkylcellulosephthalate wie Hydroxypropylmethylcellulosephthalat; Hydroxyalkylcelluloseacetatsuccinate wie Hydroxypropylmethylcelluloseacetatsuccinat; Vinylacetatphthalate; Vinylacetatsuccinat; Celluloseacetattrimelliat; Polyvinylderivate wie Polyvinylacetatphthalat, Polyvinylalkoholphthalat, Polyvinylbutylatphthalat und Polyvinylacetoacetalphthalat; Zein; Schellack; oder Gemische davon umfasst.

8. Suspensionszusammensetzung mit verlängerter Freisetzung nach Anspruch 6, wobei das pH-Wert-unabhängige Polymer aus der Gruppe ausgewählt ist, die Cellulosepolymere wie Ethylcellulose, Methylcellulose, Hydroxyethylcellulose, Hydroxypropylcellulose, Hydroxyethylmethylcellulose, Hydroxypropylmethylcellulose und Carboxymethylcellulose; Acryl-Copolymere wie Methacrylsäure-Copolymere, *z*. *B.* Eudragit^{®} RS, Eudragit^{®} RL, Eudragit^{®} NE 30 D; Celluloseacetat; Polyethylen-Derivate, *z*. *B.* Polyethylenglycol und Polyethylenoxid; Polyvinylalkohol; Polyvinylacetat; Gummis, *z*. *B.* Guargummi, Johannisbrotbaumgummi, Tragant, Carrageen, Algensäure, Akaziengummi, Gummi arabicum, Gellangummi und Xanthangummi; Triglyceride; Wachse, *z*. *B.* Compritol^{®}, Lubritab^{®} und Gelucires^{®}; Lipide; Fettsäuren oder deren Salze/Derivate; ein Gemisch aus Polyvinylacetat und Polyvinylpyrrolidon, *z*. *B.* Kollidon^{®} SR; oder Gemische davon umfasst.

9. Suspensionszusammensetzung mit verlängerter Freisetzung nach Anspruch 1, wobei die Suspensionsbasis ferner einen oder mehrere pharmazeutisch akzeptable Hilfsstoffe umfasst, die aus der Gruppe ausgewählt sind, die Suspendiermittel, Trennmittel, Netzmittel, Konservierungsmittel, Puffermittel, Aromamittel, Antioxidantien und Chelatbildner umfasst.

## Revendications

1. Composition de suspension à libération prolongée stable comprenant :
(a) de multiples noyaux enrobés comprenant :
(i) un noyau comprenant un principe actif ; et
(ii) une couche d'enrobage sur ledit noyau comprenant un ou plusieurs polymères de régulation de libération ;
(b) une base de suspension comprenant un agent osmotique, dans laquelle la base de suspension génère une condition hypertonique de sorte qu'il n'y a pas de changement substantiel dans le profil de libération par dissolution *in vitro* des compositions de suspension à libération prolongée après stockage pendant au moins sept jours, dans laquelle le principe actif est sélectionné parmi le groupe comprenant la metformine ou la guanfacine.

2. Composition de suspension à libération prolongée selon la revendication 1, dans laquelle la composition est une suspension ou une poudre reconstituée pour une suspension.

3. Composition de suspension à libération prolongée selon la revendication 1, dans laquelle le principe actif est disposé en couches sur une particule inerte pour former le noyau.

4. Composition de suspension à libération prolongée selon la revendication 3, dans laquelle la particule inerte est sélectionnée parmi le groupe comprenant un graine non pareil, une sphère de cellulose microcristalline, une perle de phosphate de calcium dibasique, une perle de mannitol, une perle de silice, une pastille d'acide tartrique, ou une pastille à base de cire.

5. Composition de suspension à libération prolongée selon la revendication 1, dans laquelle l'agent osmotique est sélectionné parmi le groupe comprenant des glucides tels que le xylitol, le mannitol, le sorbitol, l'arabinose, le ribose, le xylose, le glucose, le fructose, le mannose, le galactose, le saccharose, le maltose, le lactose, le dextrose et le raffinose ; des sels solubles dans l'eau d'acides inorganiques tels que le chlorure de magnésium, le sulfate de magnésium, le sulfate de potassium, le chlorure de lithium, le chlorure de sodium, le chlorure de potassium, l'hydrogénophosphate de lithium, l'hydrogénophosphate de sodium, l'hydrogénophosphate de potassium, le dihydrogénophosphate de lithium, le dihydrogénophosphate de sodium, le dihydrogénophosphate de potassium, et le phosphate de sodium tribasique ; des sels solubles dans l'eau d'acides organiques tels que l'acétate de sodium, l'acétate de potassium, le succinate de magnésium, le benzoate de sodium, le citrate de sodium et l'ascorbate de sodium ; des acides aminés solubles dans l'eau tels que la glycine, la leucine, l'alanine, la méthionine ; l'urée ou ses dérivés ; le propylène glycol ; la glycérine ; le polyoxyde d'éthylène ; la gomme de xanthane ; l'hydroxypropylméthyl cellulose ; ou leurs mélanges.

6. Composition de suspension à libération prolongée selon la revendication 1, dans laquelle le polymère régulant la libération est sélectionné parmi le groupe comprenant un polymère dépendant du pH, un polymère indépendant du pH, ou leurs mélanges.

7. Composition de suspension à libération prolongée selon la revendication 6, dans laquelle le polymère dépendant du pH est sélectionné parmi le groupe comprenant des copolymères acryliques tels que les copolymères d'acide méthacrylique et de méthacrylate de méthyle, *par ex.,* Eudragit^{®} L 100 et Eudragit^{®} S 100, des copolymères d'acide méthacrylique et d'acrylate d'éthyle, *par ex*. Eudragit^{®} L 100-55 et Eudragit^{®} L 30 D-55, des copolymères de méthacrylate de diméthylaminoéthyle et de méthacrylate de butyle et de méthacrylate de méthyle *par ex.,* Eudragit^{®} E 100, Eudragit^{®} E PO, des copolymères d'acrylate de méthyle et d'acide méthacrylique et d'acrylate d'octyle, des copolymères de styrène et d'acide acrylique, des copolymères d'acrylate de butyle et de styrène et d'acide acrylique, et un copolymère d'acrylate d'éthyle-acide méthacrylique ; le phtalate acétate de cellulose ; les succinates acétate de cellulose ; les phtalates d'hydroxyalkyl cellulose tels que le phtalate d'hydroxypropylméthyl cellulose ; les succinates acétate d'hydroxyalkyl cellulose tels que le succinate acétate d'hydroxypropylméthyl cellulose ; les phtalates acétate de vinyle ; le succinate acétate de vinyle ; le trimelliate acétate de cellulose ; les dérivés de polyvinyle tels que le phtalate polyacétate de vinyle, le phtalate polyalcool vinylique, le phtalate de polybutylate de vinyle et le phtalate polyacétoacétal de vinyle ; la zéine ; le vernis à la gomme laque ; ou leurs mélanges.

8. Composition de suspension à libération prolongée selon la revendication 6, dans laquelle le polymère indépendant du pH est sélectionné parmi le groupe comprenant des polymères cellulosiques tels que l'éthyl cellulose, la méthyl cellulose, l'hydroxyéthyl cellulose, l'hydroxypropyl cellulose, l'hydroxyéthylméthyl cellulose, l'hydroxypropylméthyl cellulose, et la carboxy méthylcellulose ; des copolymères acryliques tels que des copolymères d'acide méthacrylique, *par ex.,* Eudragit^{®} RS, Eudragit^{®} RL, Eudragit^{®} NE 30 D ; l'acétate de cellulose ; des dérivés de polyéthylène *par ex*., le polyéthylène glycol et le polyoxyde d'éthylène ; le polyalcool vinylique ; le polyacétate de vinyle ; des gommes *par ex.,* la gomme de guar, la gomme de caroube, l'adragante, la carraghénine, l'acide alginique, la gomme d'acacia, la gomme arabique, la gomme gellane, et la gomme de xanthane ; les triglycérides ; les cires, *e.g*., Compritol^{®}, Lubritab^{®}, et Gelucires'" ; les lipides ; les acides gras ou leurs sels/dérivés ; un mélange de polyacétate de vinyle et de polyvinyl pyrrolidone, *par ex.,* Kollidon ^{®} SR ; ou leurs mélanges.

9. Composition de suspension à libération prolongée selon la revendication 1, dans laquelle la base de suspension comprend en outre un ou plusieurs excipients pharmaceutiquement acceptables sélectionnés parmi le groupe comprenant des agents de mise en suspension, des agents anti-agglomérants, des agents mouillants, des conservateurs, des agents tampons, des agents aromatisants, des antioxydants et des agents chélateurs.
